# EUROPÄISCHE PATENTSCHRIFT

(11) **EP 3 638 198 B1**
(45) Veröffentlichungstag und Bekanntmachung des Hinweises auf die Patenterteilung: **18.08.2021**
(21) Anmeldenummer: 18734120.1
(22) Anmeldetag: 12.06.2018
(51) Int. Cl.: A61K 8/63, A61K 8/34, A61K 8/49, A61Q 7/00, A61K 31/4412, A61K 31/505

(54) **ZUSAMMENSETZUNG ZUR VERHINDERUNG VON HAARAUSFALL UND WACHSTUMSFÖRDERUNG VON HAAREN**
COMPOSITION TO PREVENT HAIRLOSS AND PROMOTE HAIRGROWTH
COMPOSITION POUR FREINER LA CHUTE DES CHEVEUX ET FAVORISER LEUR REPOUSSE

(30) Priorität: 16.06.2017 TR 201708931
(43) Veröffentlichungstag der Anmeldung: 22.04.2020
(73) Patentinhaber: Capilli Med GmbH, 24568 Oesrdorf/Hamburg (DE)
(72) Erfinder: ÖGER, Vural, 22605 Hamburg (DE); ALI KAY, Mehmet, 06670 Seyranbaglari Cankaya Ankara (TR)
(74) Vertreter: Patentanwälte Olbricht Buchhold Keulertz
(86) Internationale Anmeldenummer: PCT/EP2018/065546
(87) Internationale Veröffentlichungsnummer: WO 2018/229072

(56) Entgegenhaltungen:
- WO-A2-2007/101357
- DE-A1-102011 089 366
- US-A- 5 760 046
- US-A1- 2015 216 986
- DATABASE GNPD [Online] MINTEL; Dezember 2015 (2015-12), "Hair treatment Spray 5%", XP002784110, Database accession no. 3593763
- DATABASE GNPD [Online] MINTEL; Februar 2017 (2017-02), "Tonic Lotion", XP002784111, Database accession no. 4605291

## Beschreibung

### Stand der Technik:

Haarausfall bedeutet, dass Menschenhaare zuerst dünner werden und dann unter Umständen das gesamte Haar ausfällt. Haarausfall kann als eine Art Krankheit bewertet werden, bei der die Haare dünner und schwächer werden und deshalb ausfallen. Jeder erwachsene Mensch kann an einem Tag 50-100 Haare verlieren. Der Verlust der Haare bewirkt ernsthafte Probleme bei den Betroffenen. Das wichtigste Accessoire einer Frau oder eines Mannes sind die Haare. Schönes, volles und gepflegtes Haar zieht überall die Blicke auf sich. Es lässt sie schön, gepflegt und wirkungsvoll aussehen. Aus diesem Grund sind die Haare für das Selbstwertgefühl sehr wichtig. Wenn ein Mensch stärkeren Haarausfall erlebt, fangen psychische Probleme an.

Der medizinische Begriff für Haarausfall bei Männern und Frauen ist "Androgenetische Alopezie". Bei Männern nennt man dies zusätzlich "Männliche Kahlheit". Der Haaransatz geht mit der Zeit zurück und wird dünner. Meistens fällt dann der vordere Bereich des Haaransatzes zuerst aus. Bei Frauen hingegen tritt der Haarausfall überall gleichzeitig auf. Der männliche Haarausfall ist die am häufigsten vorkommende Art von Haarausfall. Er fängt am oberen Teil an und mit der Zeit verliert sich der vordere Haaransatz. Die Haare werden dünner. Somit kommt es zur Kahlheit in dem Bereich. Dies hält eine Weile an und es bleibt nur noch im Nacken etwas Haar übrig. In den letzten Jahren ist das Alter für Haarausfall auf 18 Jahre zurückgegangen. Der wichtigste Grund für diese Art von Haarausfall ist die Veränderung des Testosterons, welches ein androgenes Hormon ist. Testosteron verändert sich durch ein Enzym mit dem Namen 5-Alpha-Reduktase in Dihydrotestosteron (DHT). Dieses Hormon sendet Signale an das Haar und bewirkt dadurch, dass es dünner und weniger wird. Eine frühzeitige Diagnose verlangsamt oder stoppt diesen Prozess.

Haarausfall kommt bei 80 Prozent der Männer vor. Viele Frauen haben heutzutage ein Problem mit Haarausfall. Besonders Stress, falsche Ernährung, hormonale Veränderungen, Schwangerschaft, Menopause, die Benutzung falscher kosmetischer Produkte, Haarfarbe oder saisonale Bedingungen können Gründe für einen Haarausfall sein.

Haarausfall kann man in 3 Arten unterteilen. Diese sind:
1- Haarausfall am oberen Wirbel, beginnend 1-2cm hinter Stirn-Haargrenze
2- Starker Ausfall am oberen Wirbel
3- Ausfall im gesamten Kopfbereich.

In der heutigen Zeit gibt es vielerlei Gründe für einen Haarausfall.
1- Körperlicher Stress: Alle körperlichen Traumata, Operationen, schwere Krankheit, und sogar Grippe kann für vorrübergehenden Haarausfall ein Grund sein. Der Haarausfall wird meistens 3-5 Monate nach dem Ereignis sichtbar.
2- Schwangerschaft: Eine Schwangerschaft ist eine Art von körperlichem Stress, der zu Haarausfall führen kann. Schwangerschaftsbedingter Haarausfall kann während der Schwangerschaft auftreten, er kann aber auch nach der Geburt anhalten.
3- Erhöhte Vitamin A Einnahme: Laut der Amerikanischen Akademie für Dermatologie kann Vitamin A als Lebensmittelergänzung oder in Medikamenten für Haarausfall verantwortlich sein.
4- Proteinmangel: Laut der Amerikanischen Akademie für Dermatologie kann es sein, dass der Körper bei einem Proteinmangel während einer Diät den Haarwuchs stoppt, um den Mangel zu beheben.
5- Männliche Kahlheit: Bei ungefähr 2 von 3 Männern wird ab dem 60. Lebensjahr das Haar lichter. Solch ein Haarausfall hat genetische Gründe und geschieht im Zusammenhang mit den männlichen Hormonen.
6- Genetik: Der Haarausfall bei Frauen, der auch androgene oder androgenetische Alopezie genannt wird, ist im Grunde der männliche Haarausfall in seiner weiblichen Version. Wenn bei Frauen innerhalb einer Familie ab einem bestimmten Alter Haarausfall vorkommt, kann es sein, dass es erblich ist.
7- Weibliche Hormone: Wie in der Schwangerschaft können auch andere Hormone für Haarausfalt verantwortlich sein, aber auch Veränderungen im hormonellen Gleichgewicht oder die Menopause.
8- Emotionaler Stress: Es kommt seltener vor, dass emotionaler Stress der Grund für Haarausfall ist, als es bei körperlichem Stress der Fall ist. Dieser tritt meistens nach einer Scheidung oder nach dem Verlust eines geliebten Menschen vor.
9- Hypothyreose: Die Schilddrüsen, die sich am Hals befinden, ist neben den durch die produzierten Hormone hervorgerufenen Wachstums und der körperlichen Entwicklung auch für den Metabolismus verantwortlich. Wenn die Schilddrüse nicht ausreichend Hormone herstellen kann, kann dies zu Haarausfall führen.
10- Haarausfall aufgrund von Autoimmunität: Dieser Zustand ist im Grunde auf ein überaktives Immunsystem zurückzuführen. Aufgrund eines Chaos im Körper können die Haare als Fremdkörper identifiziert und bekämpft werden.
11- Lupus: Auch Lupus, eine andere Autoimmunkrankheit, kann zu Haarausfall führen.
12- Gewichtsverlust: Plötzlicher Gewichtsverlust kann für den Körper eine Art Trauma darstellen und auch zu Haarausfall führen. Haarausfall kann auch ein Zeichen von Bulimie sein.
13- Polvzystisches Ovar-Syndrom (PZOS): ist eine andere Form von Hormonstörung. Eine Überzahl von Androgenen kann zu Eierstockzysten, Gewichtszunahme, starkes Risiko für Diabetes, Änderungen in der menstrualen Periode, Unfruchtbarkeit oder Haarausdünnung führen.
14- Antidepressiva, Blutverdünner etc.: Manche Medikamentenklassen können einen Haarausfall verschlimmern. Die am häufigsten vorkommenden darunter sind einige Blutverdünner und Beta-Blocker, welche als Blutdruckmedikamente bekannt sind.
15- Übermäßiges Frisieren: Ein übermäßiges Frisieren über Jahre hinweg oder Haarbehandlungen kann zu Haarausfall führen. Zum Beispiel straffe Zöpfe oder chemische Auflockerungen, um die Haare zu glätten.
16- Altern: Bei Frauen ab dem 50. Oder 60. Lebensjahr kommt Haarausfall häufiger vor, der Grund hierfür ist noch unbekannt.
17- Anabolische Steroide: Laut der Amerikanischen Akademie für Dermatologie haben Menschen, die anabolische Steroide zu sich nehmen ein erhöhtes Risiko für Haarausfall. Anabolische Steroide haben auf den Körper die gleichen Auswirkungen wie ein Poiyzystische Ovar-Syndrom.
Bei den oben genannten Gründen für Haarausfall gehen die Haarwurzeln in einen Schlafzustand und wachsen nicht weiter.

Auf dem Markt gibt es einige pflanzliche oder medizinische Produkte, die einen Haarwuchs fördern. Einige von denen sind in TR 2016/12319 beschrieben: "Bei dieser Erfindung handelt es sich um eine Lösung, die, wenn sie in den eingestellten Proportionen von Procapil, Capixyl und Redensyl kombiniert benutzt wird vor Haarausfall schützt und zu erneutem Haarwachstum führt. Sie ist ein pflanzliches unterstützendes Mittel, das im Gegensatz zu vielen pflanzlichen Mitteln für die Verhinderung von Haarausfall stärker und wirkungsvoller ist, wenn es in einer geeigneten Konzentration benutzt und kombiniert wird."

In einem anderen Patentanmeldung mit der Nummer TR 2014/05932 heißt es "Es handelt sich um eine Komposition aus Talg, Gelbwurzel, Kienteer, Olivenöl, Vaseline und Wachs für die Verhinderung von Haarausfalt/das erneute Haarwachstum, und die Herstellungsmethoden dieser Komposition." Es gibt auf dem Markt viele ähnliche Produkte, wie die, die oben beschrieben sind.

DE 10 2011 089366 A1 offenbart ethanolische Extrakte von Moringa oleifera, welche Minoxidil und Finasterid enthaltend.

### Beschreibung der Erfindung

Gegenstand der Erfindung ist eine alkoholische Lösung zur Verhinderung von Haarausfall und für erneutes Haarwachstum gemäß Patentanspruch 1. Weiterhin ist Gegenstand der Erfindung ein Verfahren zur Herstellung der alkoholischen Lösung gemäß Patentanspruch 6.

Weitere Ausführungsformen sind Gegenstand der Unteransprüche oder nachfolgend beschrieben.

Die erfindungsgemäße alkoholische Lösung ist ein Haarwuchsmittel, das topisch angewendet wird. Die alkoholische Lösung enthält Ethanol, Monopropylenglykol, Minoxidil, Finasterid, Vitamin B2 und Koffein. Sie ist bevorzugt frei von Wasser.

Die erfindungsgemäße alkoholische Lösung enthält bevorzugt
- 10 bis 30 Gew.-%, bevorzugt 15 bis 30 Gew.-%, besonders bevorzugt 17 bis 27 Gew.-% Ethanol,
- 60 - 85 Gew.-%, bevorzugt 65 - 80 Gew.-%, besonders bevorzugt 65 bis 75 Gew.-% Monopropylenglykol,
- 2 bis 7,5 Gew.%, bevorzugt 3 bis 6 Gew.-% Minoxidil und
- 0,1 bis 3 Gew.-%, bevorzugt 0,1 bis 2,7 Gew.-%, besonders bevorzugt 0,1 bis 0,5 Gew.-% Finasterid,
- 0,001 - 2 Gew.-%, bevorzugt 0,001 bis 0,5 Gew.-% Vitamin B2,
- 0,0005 bis 2 Gew.-% Koffein, jeweils bezogen auf das Gesamtgewicht der alkoholischen Lösung.

Die erfindungsgemäße alkoholische Lösung enthält besonders bevorzugt
- 17 bis 27 Gew.-% Ethanol,
- 65 bis 75 Gew.-% Monopropylenglykol,
- 3 bis 6 Gew.-% Minoxidil und
- 0,1 bis 0,5 Gew.-% Finasterid,
- 0,001 bis 0,5 Gew.-% Vitamin B2,
- 0,0005 bis 2 Gew.-% Koffein, jeweils bezogen auf das Gesamtgewicht der alkoholischen Lösung.

Die erfindungsgemäße Lösung enthält Vitamin B2, bevorzugt 0,001 - 2 Gew.-%, bevorzugt 0,001 bis 0,5 Gew.-% Vitamin B2, jeweils bezogen auf das Gesamtgewicht der Lösung.

Die Lösung enthält Koffein, bevorzugt 0,0005 bis 2 Gew.-% Koffein, bezogen auf das Gesamtgewicht der Lösung. Optional enthält die Lösung zusätzlich Penisetum Glaucum.

In einer bevorzugten Ausführungsform besteht die erfindungsgemäße Lösung aus Ethanol, Monopropylenglykol, Finasterid, Minoxidil, Vitamin B2 und Koffein und enthält keine weitere Bestandteile. Bevorzugt besteht die alkoholische Lösung aus
- 10 bis 30, bevorzugt 15 bis 30 Gew.-%, besonders bevorzugt 17 bis 27 Gew.-% Ethanol,
- 60 - 85, bevorzugt 65 - 80 Gew.-%, besonders bevorzugt 65 bis 75 Gew.-% Monopropylenglykol,
- 2 bis 7,5 Gew.%, bevorzugt 3 bis 6 Gew.-% Minoxidil und
- 0,1 bis 3 Gew.-%, bevorzugt 0,1 bis 2,7 Gew.-%, besonders bevorzugt 0,1 bis 0,5 Gew.-% Finasterid,-
- 0,001-2 Gew.-% Vitamin B2
- 0,0005-2 Gew.-% Koffein und
- 0-2 Gew.-% Penisetum Glaucum,
wobei sich die Komponenten zu 100 Gew.-% ergänzen.

Die erfindungsgemäße alkoholische Lösung ist ein Mittel zur Verhinderung von Haarausfall und für das erneute Haarwachstum; die aufgrund von Haarausfall unter der Kopfhaut schlafenden Haarwurzeln werden angeregt, erneut zu wachsen.

Die erfindungsgemäße alkoholische Lösung regt überraschend mit der enthaltenen Mischung die Wurzeln an, erneut Haare zu produzieren, indem sie sie mit Nährstoffen versorgt.

Die erfindungsgemäße alkoholische Lösung bewirkt durch die einfache Benutzung und dadurch, dass sie keine Nebenwirkungen hat, dass dem Anwender wieder Haare wachsen. Die erfindungsgemäße alkoholische Lösung kann außer bei Frauen in einer Risikoschwangerschaft gut genutzt werden, da sie sehr verträglich ist. Das optional in der erfindungsgemäße alkoholische Lösung enthaltene Vitamin B2 bewirkt, dass die Haarwurzeln wieder aufwachen, genährt werden und wieder wachsen. Die erfindungsgemäße alkoholische Lösung reinigt aufgrund ihrer antioxidanten Wirkung die Zellen und hilft den Haaren, wieder gesund zu wachsen. Die erfindungsgemäße alkoholische Lösung ernährt die Haare nach dem Wachstum und unterstützt eine gesunde Haarpracht.

Diese erfindungsgemäße alkoholische Lösung bewirkt, dass die in der Kopfhaut ruhenden Haarwurzeln nach dem Haarausfall wieder stimuliert werden und wieder wachsen; nach einer Benutzung für eine bestimmte Zeit kehren die Haare bleibend wieder; außerdem werden die Haarwurzeln hiermit mit Vitaminen versorgt und ein Haarausfall wird verhindert.

Die erfindungsgemäße alkoholische Lösung enthält Alkohol, Monopropylenglykol, Minoxidil und Finasterid und Vitamin B2 und Koffein.

Ethanol: Wird in vielen Sektoren genutzt und wird in der Kosmetik- und Medizinbranche als Lösungsmittel genutzt. Es hat in variablen Nutzungsgebieten verschiedene Namen. Erfindungsgemäß wird Ethanol 96 % verwendet. Alle Gewichtsprozentangaben beziehen sich auf 96 % Ethanol und nicht auf 100 % Ethanol.

Monopropylenglykol: Ist ein Lösungsmittel mit geringer Flüchtigkeit, kann mit Wasser, Alkohol oder Ester gemischt werden und ist mit hoher Reinheit, durchsichtig und farblos. Es ist besonders für kosmetische Produkte geeignet. Es ist neutral und hygroskopisch.

Minoxidil: Ist das erste Medikament, das von der FDA lizenziert wurde, um Haarausfall zu verhindern. Minoxidil ist eigentlich ein oral verabreichtes, vasodilatatorisches Medikament zur Blutdruckverringerung, das eine Verengung der Blutgesäße bewirkt. Minoxidil bewirkt bei schon vorhandenen kleinen Haarfollikeln ein Zurückkehren zu dem normalen Umfang und eine Hypertrophie. Eine Rückkehr oder Bildung von neuen Haarfollikeln konnte bei den bekannten Präparaten nicht beobachtet werden.

Finasterid: wurde im Jahre 1992 unter dem Namen Propecia herausgebracht, um eine Prostatavergrößerung zu verhindern.

Testosteron ist ein Alpha 5 Reduktase Enzym, dass eine Umformung des Dihydrotestosterons bewirkt, welches auf die Haarwurzeln wirkt und zu Haarausfall führt. Dieses Enzym wird durch Finasterid in seiner Aktivität gehemmt sofern Finasterid täglich oral eingenommen wurde.

Vitamin B2: Vitamin B2, das auch Riboflavin heißt, spielt in der Umwandlung von Kohlehydraten und Fetten in Energie eine Rolle. Es wurde zum ersten Mal in 1933 aus Milch isoliert. Vitamin B2 ist ein wasserlösliches Vitamin und robust gegen Sonneneinstrahlung und Alkalie.

Koffeine: Trimethylxanthin. Ist ein Alkaloid, das in Kaffee, Tee, Limonaden, Kakao und Kakaoprodukten vorkommt. Außerdem findet man es auch in krampflösenden, abschwellenden und antiallergischen Mitteln. Bei dieser Erfindung wird es als Stimulans in einer topische Anwendung genutzt.

Die erfindungsgemäße alkoholische Lösung verhindert überraschenderweise Haarausfall nicht nur, sondern bewirkt einen erneuten Haarwuchs d.h. eine Neuhaarbildung in bereits haarlosen Bereichen.

Durch die erfindungsgemäße alkoholische Lösung werden
- schlafenden Haarwurzeln stimuliert,
- Haarwurzeln am Leben gehalten,
- Hautporen geöffnet, damit die Wirkstoffe in die Haut eindringen können,
- die Haarwurzeln ernährt und gestärkt und
- erreicht, dass die Haare genährt und gesund sind.

Gegenstand der Erfindung ist weiterhin die erfindungsgemäße alkoholische Lösung zur Verwendung als topisches Haarwuchsmittel, zum Auftragen auf die Haut.

Die erfindungsgemäße Verwendung der alkoholischen Lösung als topisches Haarwuchsmittel, insbesondere zur Verhinderung von Haarausfall und zur Wachstumsförderung von Haaren ist besonders vorteilhaft. Die alkoholische Lösung wird hierzu auf die Haut auf die haarlosen Bereiche aufgetragen.

### Herstellung der Lösung:

Die erfindungsgemäße alkoholische Lösung wird aus mehreren Mischungen (Phasen) hergestellt.

### Phase 1:

A) Es wird eine Mischung aus 10-30 Gew.-% Ethanol in ein Reaktionsgefäß gegeben.
B) 0,1 bis 3 Gew.-% Finasterid wird hinzugefügt und im Alkohol vollständig aufgelöst.
C) Die entstandene Mischung (Phase 1) wird nach der vollständigen Auflösung zum Ruhen zur Seite gestellt. Bevorzugt ruht die Mischung für 1 bis 60 min.

### Phase 2:

A) Zunächst wird 60 - 85 Gew.-% Monopropylenglykol vorgelegt und auf 25 - 45 °C erwärmt.
B) Darauf wird Minoxidil zu 2 bis 7,5 Gew.-% der Gesamtmischung hinzugefügt und in Monopropylenglykol vollständig aufgelöst. Hierzu wird die Mischung bei 25-45 °C bevorzugt für ca. 2 - 5 h gerührt.
C) Die Mischung (Phase 2) wird nach der vollständigen Auflösung bevorzugt zum Ruhen zur Seite gestellt. Bevorzugt ruht die Mischung für 15 bis 60 min.

Zu Phase 1 werden weitere Stoffe zugegeben:
A) Zunächst wird die Mischung aus Alkohol und Finasterid (Phase 1) in einen Kessel gegeben.
B) Darauf wird Koffein zu 0,0005%-2% gegeben und vollständig aufgelöst.
C) Darauf wird Vitamin B2 zu 0,001%-2% gegeben und vollständig aufgelöst.
D) Die Mischung wird nach der vollständigen Auflösung bevorzugt zum Ruhen zur Seite gestellt.
A) Für die Herstellung der alkoholischen Lösung wird Phase 2 Phase 1 hinzugefügt und die Phasen gemischt. Bevorzugt ruht die Mischung für 15 bis 60 min.

Das erfindungsgemäße Verfahren wird bevorzugt zur Herstellung der erfindungsmäßen alkoholischen Lösung eingesetzt. Entsprechend werden die einzelnen Komponenten beim erfindungsgemäßen Verfahren in den vorstehenden Mengen eingesetzt.

Das erfindungsgemäße Verfahren zur Herstellung einer alkoholischen Lösung enthaltend Ethanol, Monopropylenglykol, Finasterid, Minoxidil, Vitamin B2 und Koffein, umfasst die Schritte
A. Herstellen einer ersten Phase über die Verfahrensschritten
   A.1. Vorlegen von Ethanol,
   A.2. Zugeben von Finasterid in die Lösung gemäß A.1. und Rühren bis sich Finasterid aufgelöst hat und eine klare Lösung entstanden ist (Phase 1),
   A.3. Zugeben von 0,0005%-2% Koffein und rühren bis es vollständig aufgelöst ist,
   A.4. Zugeben von 0,001 %-2% Vitamin B2 und rühren bis es vollständig aufgelöst ist und
   ggf. A5 Ruhen lassen der Mischung aus Schritt A.2, A.3 oder A.4,
B. Herstellen einer zweiten Phase über die Verfahrensschritten
   B.1. Vorlegen von Monopropylenglykol in einem Gefäß,
   B.2. Erwärmen der Mischung auf Temperaturen von 25 bis 45 °C, bevorzugt 30 bis 40°C,
   B.3. Zugeben von Minoxidil in die Lösung und rühren bis sich Minoxidil aufgelöst hat (Phase 2),
   ggf. B.4. Ruhen lassen der Mischung aus Schritt B.3,
C. Herstellen der endgültigen Lösung über die Verfahrensschritten
   C.1. Vorlegen von Phase 1 oder Phase 2, bevorzugt Phase 1,
   C.2. Zugeben von Phase 2 in Phase 1 oder Phase 1 in Phase 2 und mischen.
   ggf. C.3. Ruhen lassen der Mischung aus Schritt C.2.

Beim Herstellen der ersten Phase gemäß Schritt A wird die Mischung in Schritt A.2. bevorzugt 1 bis 10 min gerührt, bevorzugt 2 bis 6 min. Optional wird die Mischung gemäß Phase 1 beim Rühren ebenfalls auf Temperaturen von 25 bis 45 °C, bevorzugt 30 bis 40°C erwärmt. Ein Ruhen gemäß Schritt A.5. dauert bevorzugt 1 bis 60 min, besonders bevorzugt 1 bis 15 min.

Beim Herstellen der zweiten Phase gemäß Schritt B wird die Mischung in Schritt B.3. bevorzugt 2 bis 5 h gerührt, bevorzugt 2,5 bis 4,5 h. Die Mischung gemäß Schritt B.4 wird bevorzugt für 15 bis 60 min, bevorzugt 20 bis 45 min, ruhen gelassen.

In Phase 1 wird Finasterid in Ethanol gegeben und aufgelöst,
- Damit werden die schlafenden Haarwurzeln stimuliert,
- Damit werden die Haarwurzeln am Leben gehalten,
- Damit werden die Hautporen geöffnet, damit das Finasterid und ggf. Vitamin eindringen kann.

In Phase 2 wird Minoxidil in Monopropylenglykol aufgelöst,
- Damit werden die schlafenden Haarwurzeln stimuliert,
- Damit werden die Haarwurzeln am Leben gehalten,
- Damit werden die Hautporen geöffnet, damit das Minoxidil eindringen kann.

Die zusätzliche Verwendung von Koffein und Vitamin B2 in der Mischung sorgt ebenfalls dafür,
- dass die schlafenden Haarwurzeln stimuliert werden,
- dass die Haarwurzeln ernährt und gestärkt werden,
- dass bewirkt wird, dass die Haare genährt und gesund sind.

Die erfindungsmäße Lösung aus Ethanol, Monopropylenglykol, Minoxidil, Finasterid und Vitamin B2 und Koffein wird topisch angewendet. Die alkoholische Lösung wird direkt auf die Haut in den haarlosen bzw. zu behandelnden Bereichen aufgetragen. Zunächst wird hierfür der Anwendungsbereich gesäubert und getrocknet. Es werden einige Tropfen der erfindungsgemäßen alkoholischen Lösung auf die haarlosen Bereiche aufgetragen. Bevorzugt werden die Tropfen mit den Fingern im haarlosen Bereich einmassiert. Es folgt bevorzugt eine Massage für z.B. etwa 1 - 10, bevorzugt 5 bis 10 Minuten. Während Ethanol sich verflüchtigt, dringen Minoxidil, Finasterid, Vitamin B2 und das Koffein in die Haut ein und stimulieren die schlafenden Haarwurzeln und bewirken so, dass sie wiederwachsen. Die Mischung nährt die neu wachsenden Haare und ihre Wurzeln.

In einer Ausführungsform der Erfindung beträgt die Anwendungsdauer beträgt in etwa ein Jahr. Die Anwendung erfolgt bevorzugt innerhalb der ersten 6 Monate jeden Morgen und jeden Abend. Am Ende der ersten 6 Monate wird dann bevorzugt je nach Haarwachstum in wöchentlichen, monatlichen oder 2-monatlichen Phasen bis zum Ende der zweiten 6-monatigen Periode die Behandlung fortgeführt. Nachdem das gesamte Haarwachstum wiederhergestellt wurde, wird die Behandlung bevorzugt in bestimmten Abständen fortgeführt, um sicherzustellen, dass die Haare und ihre Wurzeln gut genährt werden und um einen erneuten Haarausfall zu verhindern.

Minoxidil und Finasterid werden bei dieser Erfindung zum ersten Mal überhaupt in einem Mittel zusammen verwendet und erstmalig topisch angewandt. Überraschend führt die Kombination von Minoxidil und Finasterid dazu dass, wie oben schon beschrieben, die Haarwurzeln stimuliert werden und bewirken, dass die Haare wieder wachsen. Außerdem bewirkt auch das in dieser Lösung verwendete Vitamin B2, dass die Haare und Haarwurzeln sich gesund entwickeln und die Haare wieder wachsen, außerdem dass die Haare und Wurzeln gut genährt werden. Das in der Lösung enthaltene Monopropylenglykol vernichtet die Bakterien und unterbindet die Entstehung von Bakterien.

Zusätzlich zu dem sich in der Lösung befindenden Minoxidil und Finasterid wird der alkoholischen Lösung Koffein hinzugefügt, dass die gleiche Wirkung hat. Somit werden die Wurzeln stimuliert und ein erneutes Haarwachstum unterstützt. Wenn die erfindungsgemäße alkoholische Lösung regelmäßig angewandt wird, bewirkt sie ein erneutes Haarwachstum Im kahlgewordenen Bereich.

Bei ersten Tests wurde eine erfindungsgemäße alkoholische Lösung enthaltend Ethanol, Monopropylenglykol, Minoxidil, Finasterid, Vitamin B2 und Koffein verwendet. Die Lösung wurde bei 10 männlichen Probanden über 6 Monate angewendet. Die Behandlung erfolgte jeweils morgens und abends. Zunächst wurde der Anwendungsbereich gesäubert und getrocknet. Mit Hilfe einer Pipette wurde eine kleine Menge der erfindungsgemäßen alkoholischen Lösung aus einer Flasche entnommen und auf die haarlosen Bereiche tropfenweise aufgetragen. Mit den Fingern wurden die Tropfen im haarlosen Bereich einmassiert. Es folgt eine Massage für 2 bis 3 Minuten.

Nach 6 -8 Monaten Anwendungsdauer konnte bei allen Probanden eine Rückkehr von Haaren im vorher haarlosen Bereich (Neuhaarbildung) und eine Stärkung des Haarwuchs in den umgebenden Bereichen beobachtet werden.

## Patentansprüche

1. Alkoholische Lösung enthaltend
- Ethanol,
- Monopropylenglykol,
- Minoxidil und
- Finasterid
**dadurch gekennzeichnet, dass**, die Lösung
zusätzlich Vitamin B2 und/oder zusätzlich Koffein enthält.

2. Lösung gemäß Patentanspruch 1, **dadurch gekennzeichnet, dass** die Lösung enthält
- 10 bis 30 Gew.-%, bevorzugt 15 bis 30 Gew.-% Ethanol,
- 60 - 85 Gew.-%, bevorzugt 65 - 80 Gew.-% Monopropylenglykol,
- 2 bis 7,5 Gew.%, bevorzugt 3 bis 6 Gew.-% Minoxidil und
- 0,1 bis 3 Gew.-%, bevorzugt 0,1 bis 2,7 Gew.-% Finasterid,
- 0,001 - 2 Gew.-%, bevorzugt 0,001 bis 0,5 Gew.-% Vitamin B2,
- 0,0005 bis 2 Gew.-% Koffein, jeweils bezogen auf das Gesamtgewicht der Lösung.

3. Lösung gemäß einem der vorhergehenden Patentansprüche, **dadurch gekennzeichnet dass**, die Lösung besteht aus
10 bis 30 Gew.-%, bevorzugt 15 bis 30 Gew.-%, besonders bevorzugt 17 bis 27 Gew.-% Ethanol,
- 60 - 85 Gew.-%, bevorzugt 65 - 80 Gew.-%, besonders bevorzugt 65 bis 75 Gew.-% Monopropylenglykol,
- 2 bis 7,5 Gew.%, bevorzugt 3 bis 6 Gew.-% Minoxidil und
- 0,1 bis 3 Gew.-%, bevorzugt 0,1 bis 2,7 Gew.-%, besonders bevorzugt 0,1 bis 0,5 Gew.-% Finasterid,-
- 0,001-2 Gew.-% Vitamin B2
- 0,0005-2 Gew.-% Koffein und
- 0-2 Gew.-% Penisetum Glaucum,
wobei sich die Komponenten zu 100 Gew.-% ergänzen.

4. Verfahren zur Herstellung einer alkoholischen Lösung enthaltend
- Ethanol,
- Monopropylenglykol,
- Finasterid,
- Minoxidil,
umfassend die Schritte
A. Herstellen einer ersten Phase über die Verfahrensschritten
A.1. Vorlegen von Ethanol,
A.2. Zugeben von Finasterid in die Lösung gemäß A.1. und Rühren bis sich Finasterid aufgelöst hat und eine klare Lösung entstanden ist (Phase 1),
A.3. Zugeben von 0,0005%-2% Koffein und rühren bis es vollständig aufgelöst ist,
A.4. Zugeben von 0,001%-2% Vitamin B2 und rühren bis es vollständig aufgelöst und
ggf. A5 Ruhen lassen der Mischung aus Schritt A.2, A.3 oder A.4,
B. Herstellen einer zweiten Phase über die Verfahrensschritten
B.1. Vorlegen von Monopropylenglykol in einem Gefäß,
B.2. Erwärmen der Mischung auf Temperaturen von 25 bis 45 °C, bevorzugt 20 bis 40°C,
B.3. Zugeben von Minoxidil in die Lösung und rühren bis sich Minoxidil aufgelöst hat (Phase 2),
ggf. B.3. Ruhen lassen der Mischung aus Schritt B.3,
C. Herstellen der endgültigen Lösung über die Verfahrensschritten
C.1. Vorlegen von Phase 1 oder Phase 2, bevorzugt Phase 1,
C.2. Zugeben von Phase 2 in Phase 1 oder Phase 1 in Phase 2 und mischen.
ggf. C.3. Ruhen lassen der Mischung aus Schritt C.2.

5. Alkoholische Lösung gemäß einem der Patentansprüche 1 bis 3 zur Verwendung als topisches Haarwuchsmittel zum Auftragen auf die Haut.

6. Nicht-therapeutische Verwendung der alkoholischen Lösung gemäß einem der Patentansprüche 1 bis 3 als topisches Haarwuchsmittel, insbesondere zur Verhinderung von Haarausfall und zur Wachstumsförderung von Haaren.

## Claims

1. Alcoholic solution containing:
- Ethanol,
- Monopropylene glycol,
- Minoxidil, and,
- Finasteride,
**characterized in that** the solution additionally contains vitamin B2 and additionally contains caffeine.

2. Solution according to claim 1, **characterized in that** the solution contains:
- 10 to 30% by weight ethanol, preferably 15 to 30% by weight ethanol,
- 60-85% by weight monopropylene glycol, preferably 65-80% by weight monopropylene glycol,
- 2 to 7.5% by weight minoxidil, preferably 3 to 6% by weight minoxidil,
- 0.1 to 3% by weight finasteride, preferably 0.1 to 2.7% by weight finasteride, 0.001 -2% by weight vitamin B2, preferably 0.001 -0.5% vitamin B2,
- 0.0005 - 2% by weight caffeine,
each based on the total weight of the solution.

3. Solution according to one of the preceding claims, **characterized in that** the solution comprises:
10 to 30% by weight ethanol, preferably 15 to 30% by weight ethanol, particularly preferably 17 to 27% by weight ethanol,
- 60-85% by weight monopropylene glycol, preferably 65-80% by weight monopropylene glycol, particularly preferably 65 to 75% by weight monopropylene glycol,
- 2 to 7.5% by weight minoxidil, preferably 3 to 6% by weight minoxidil, and,
- 0.1 to 3% by weight finasteride, preferably 0.1 to 2.7% by weight finasteride, particularly preferably 0.1 to 0.5% by weight finasteride,
- 0.001-2% by weight vitamin B2,
- 0.0005-2% by weight caffeine, and,
- 0-2% by weight Penisetum glaucum,
the components adding up to 100% by weight.

4. Method for preparing an alcoholic solution containing:
- Ethanol,
- Monopropylene glycol,
- Finasteride,
- Minoxidil,
comprising the steps:
A. Producing a first phase using the method steps:
A.1. Providing ethanol,
A.2. Adding finasteride to the solution according to A.1. and stirring until finasteride has dissolved and a clear solution has formed (phase 1),
A.3. Adding 0.0005%-2% caffeine and stirring until completely dissolved,
A.4. Adding 0.001 %-2% vitamin B2 and stirring until completely dissolved, and,
optionally, A5 Letting the mixture from step A.2, A.3, or A.4 rest,
B. Producing a second phase using the method steps:
B.1. Placing monopropylene glycol in a vessel,
B.2. Heating the mixture to temperatures of 25 to 45°C, preferably 20 to 40°C,
B.3. Adding minoxidil to the solution and stirring until minoxidil has dissolved (phase 2),
optionally B.3. Letting the mixture from step B.3 rest,
C. Producing the final solution using the method steps:
C.1. Providing phase 1 or phase 2, preferably phase 1,
C.2. Adding phase 2 to phase 1 or phase 1 to phase 2 and mixing.
optionally C.3. Letting the mixture from step C.2 rest.

5. Alcoholic solution according to one of claims 1 to 3 for use as a topical hair restorative for application to the skin.

6. Non-therapeutic use of the alcoholic solution according to one of claims 1 to 3 as a topical hair restorative, in particular to prevent hair loss and to promote hair growth.

## Revendications

1. Solution alcoolique contenant
- de l'éthanol,
- du monopropylène glycol,
- du minoxidil et
- du finastéride
**caractérisée en ce que** la solution contient également de la vitamine B2 et/ou de la caféine.

2. Solution selon la revendication 1, **caractérisée en ce que** la solution contient
- 10 à 30 % en poids, de préférence 15 à 30 % en poids d'éthanol,
- 60 à 85 % en poids, de préférence 65 à 80 % en poids de monopropylène glycol,
- 2 à 7,5 % en poids, de préférence 3 à 6 % en poids de minoxidil et
- 0,1 à 3 % en poids, de préférence 0,1 à 2,7 % en poids de finastéride,
- 0,001 à 2 % en poids, de préférence 0,001 à 0,5 % en poids de vitamine B2,
- 0,0005 à 2 % en poids de caféine,
respectivement par rapport au poids total de la solution.

3. Solution selon l'une des revendications précédentes, **caractérisée en ce que** la solution est constituée
- de 10 à 30 % en poids, de préférence de 15 à 30 % en poids, de manière particulièrement préférée de 17 à 27 % en poids d'éthanol,
- de 60 à 85 % en poids, de préférence de 65 à 80 % en poids, de manière particulièrement préférée de 65 à 75 % en poids de monopropylène glycol,
- de 2 à 7,5 % en poids, de préférence de 3 à 6 % en poids de minoxidil et
- de 0,1 à 3 % en poids, de préférence de 0,1 à 2,7 % en poids, de manière particulièrement préférée de 0,1 à 0,5% en poids de finastéride,
- de 0,001 à 2 % en poids de vitamine B2,
- de 0,0005 à 2 % en poids de caféine et
- de 0 à 2 % en poids de Pennisetum glaucum,
les constituants se complétant jusqu'à 100 % en poids.

4. Procédé de préparation d'une solution alcoolique contenant
- de l'éthanol,
- du monopropylène glycol,
- du finastéride,
- du minoxidil,
comprenant les étapes
A. de préparation d'une première phase par les étapes de procédé
A.1. de présentation d'éthanol,
A.2. d'ajout de finastéride à la solution selon A.1. et d'agitation jusqu'à ce que le finastéride soit dissous et qu'une solution limpide se soit formée (phase 1),
A.3. d'ajout de 0,0005 % à 2 % de caféine et d'agitation jusqu'à sa dissolution complète,
A.4. d'ajout de 0,001 % à 2 % de vitamine B2 et d'agitation jusqu'à sa dissolution complète et
éventuellement A.5. de repos du mélange issu des étapes A.2, A.3 ou A.4,
B. de préparation d'une seconde phase par les étapes de procédé
B.1. de présentation du monopropylène glycol dans un récipient,
B.2. de réchauffement du mélange à des températures de 25 à 45 °C, de préférence de 20 à 40 °C,
B.3. d'ajout de minoxidil à la solution et d'agitation jusqu'à ce que le minoxidil soit dissous (phase 2),
éventuellement B.3. de repos du mélange issu de l'étape B.3,
C. de préparation de la solution finale par les étapes de procédé
C.1. de présentation de la phase 1 ou de la phase 2, de préférence de la phase 1,
C.2. d'ajout de la phase 2 dans la phase 1 ou de la phase 1 dans la phase 2 et de mélange.
éventuellement C.3. de repos du mélange issu de l'étape C.2.

5. Solution alcoolique selon l'une des revendications 1 à 3 destinée à être utilisée comme moyen de pousse topique des cheveux à appliquer sur la peau.

6. Utilisation non thérapeutique de la solution alcoolique selon l'une des revendications 1 à 3 comme moyen de pousse topique des cheveux, en particulier pour prévenir la chute des cheveux et favoriser la pousse des cheveux.
